# FASCICULE DE BREVET EUROPEEN

(11) **EP 4 129 375 B1**
(45) Date de publication et mention de la délivrance du brevet: **21.02.2024**
(21) Numéro de dépôt: 22183814.7
(22) Date de dépôt: 08.07.2022
(51) Int. Cl.: A61M 16/00

(54) **APPAREIL À TOUX AVEC AFFICHAGE DES VOLUMES DE GAZ INSUFFLÉ ET CIBLE**
HUSTENGERÄT MIT ANZEIGE DES EINGEBLASENEN GASVOLUMENS UND DES ZIELWERTS
COUGH ASSIST MACHINE WITH DISPLAY OF INHALED AND TARGET VOLUMES OF GAS

(30) Priorité: 04.08.2021 FR 2108487
(43) Date de publication de la demande: 08.02.2023
(73) Titulaire: EOVE, 64000 Pau (FR); Air Liquide Medical Systems, 92182 Antony Cedex (FR)
(72) Inventeur: LEROUX, Jean, 92182 Antony Cedex (FR); GALBRUN, Marie-Amédée, 64000 Pau (FR)
(74) Mandataire: Air Liquide

(56) Documents cités:
- EP-A1- 1 502 619
- WO-A1-2013/140333
- WO-A1-2017/032882
- WO-A2-2012/085787
- US-A1- 2007 199 566

## Description

La présente invention concerne un appareil d'assistance à la toux ou appareil à toux permettant d'opérer au moins des insufflations de gaz à un patient souffrant de troubles respiratoires nécessitant une aide pour évacuer de ses sécrétions pulmonaires et/ou pour améliorer son élasticité pulmonaire et thoracique, que ce patient soit un patient adulte ou pédiatrique, lequel peut être utilisé facilement par une personne n'ayant pas ou peu de connaissances médicales, typiquement une auxiliaire de vie, une personne de l'entourage familial du patient, encore appelés « les aidants ».

Les dispositifs médicaux appelés « assistants de toux », « appareils d'assistance à la toux », « appareil à toux » ou analogue, sont des appareils complémentaires aux respirateurs médicaux, qui permettent de générer une pression gazeuse contrôlée afin d'aider un patient à tousser, expectorer et se désencombrer.

Le principe d'un assistant de toux est d'aider à la mobilisation et à l'expectoration des sécrétions bronchiques en gonflant les poumons avec une pression positive puis en appliquant une pression négative pour faciliter le déplacement des mucosités. De tels appareils assistants de toux sont notamment décrits par EP-A-3622991, EP-A-3622992 et EP-A-862922. Un assistant de toux peut aussi être utilisé pour tenter d'améliorer l'élasticité pulmonaire et thoracique, chez certains patients.

Les dispositifs d'assistance à la toux comprennent généralement une turbine motorisée, aussi appelée compresseur ou micro-soufflante, tournant à vitesse fixe ou variable, couplée à une ou plusieurs électrovannes permettant d'orienter le flux d'air depuis la turbine vers le patient, lors des phases d'insufflation de gaz, puis depuis le patient vers la turbine, lors des phases d'exsufflation. Le patient est donc soumis à une alternance de phases d'insufflation (IN) et d'exsufflation (EX) de gaz.

Lors des phases d'insufflation, la sortie de turbine est connectée fluidiquement au patient, alors que, lors des phases d'exsufflation, l'entrée d'air de la turbine est connectée fluidiquement au patient afin d'assurer une aspiration, voire une dépression dans certains cas. Des capteurs de pression et de débit coopérant avec des moyens de pilotage permettent de gérer les différentes phases en fonction des réglages choisis, en particulier des modes ventilatoires. Un oscillateur de gaz peut également être prévu pour augmenter la capacité du dispositif à mobiliser les sécrétions pulmonaires ou bronchiques des patients traités.

La manipulation de ces appareils est habituellement confiée à un professionnel de santé, principalement les kinésithérapeutes. Ceci implique donc un déplacement systématique du professionnel de santé au domicile de chaque patient, jusqu'à plusieurs fois par jour, lorsque plusieurs séances quotidiennes sont nécessaires. De plus, le traitement du patient ne se fait pas forcément au meilleur moment pour lui car il dépend essentiellement du passage du professionnel de santé. Il serait donc souhaitable de pouvoir déclencher une séance d'utilisation de l'appareil à toux au meilleur moment pour le patient de manière à la rendre plus efficace et par ailleurs à réduire considérablement le stress du patient et à éviter des déplacements pouvant être fréquents du personnel de santé.

De plus, il peut être compliqué pour certains patients, notamment les patients pédiatriques (i.e. enfants...), d'utiliser correctement l'appareil à toux lorsqu'un kinésithérapeute ou analogue n'est pas présent pour les aider, car ils doivent réussir à synchroniser leur respiration avec les phases d'insufflation et d'exsufflation de gaz par la turbine de l'appareil à toux, pour que le traitement soit efficace.

D'autres exemples d'appareil d'assistance respiratoires permettant d'opérer des insufflations et/ou exsufflations de gaz à un patient sont enseignés par US-A-2007/199566, WO-A-2012/085787 et EP-A-1502619.

Autrement dit, le problème est de proposer un appareil d'assistance à la toux pouvant fonctionner simplement et être mis en oeuvre par une personne moins formée qu'un kinésithérapeute, par exemple une auxiliaire de vie, une personne de l'entourage familial du patient... sans avoir à faire venir spécialement un professionnel de santé qualifié de type kinésithérapeute ou analogue, tout en obtenant un traitement aussi efficace qu'en présence d'un tel kinésithérapeute et/ou en rendant le patient adhérent ou plus adhérent à son traitement, en particulier dans le cas où il convient d'améliorer l'élasticité pulmonaire et/ou thoracique du patient.

L'invention concerne alors un appareil à toux, i.e. un appareil d'assistance à la toux, pour opérer au moins une insufflation de gaz à un patient, comprenant une turbine motorisée en communication fluidique avec un circuit de gaz comprenant une ligne d'insufflation, un écran d'affichage et des moyens de pilotage configurés pour commander la turbine et/ou un affichage sur l'écran d'affichage.

Selon l'invention, les moyens de pilotage sont en outre configurés pour :
a) commander la turbine pour insuffler du gaz au patient pendant plusieurs phases d'insufflation initiales successives,
b) déterminer les volumes de gaz insufflés (Vi) au patient pendant lesdites plusieurs phases d'insufflation initiales successives,
c) déterminer au moins un volume de gaz moyen (Vm) à partir des volumes de gaz insufflés (Vi) pendant lesdites plusieurs phases d'insufflation initiales successives,
d) déterminer ou fixer un volume de gaz cible (Vc) à administrer au patient à partir du volume de gaz moyen (Vm), tel que : Vc ≥ Vm,
e) après avoir déterminé ou fixé le volume de gaz cible (Vc), commander la turbine pour fournir du gaz au patient, pendant au moins une phase d'insufflation supplémentaire suivant lesdites phases d'insufflation initiales, et
f) commander un affichage sur l'écran d'affichage, pendant chaque phase d'insufflation supplémentaire, d'au moins le volume de gaz cible (Vc) et le volume de gaz insufflé (Vi) administré au patient pendant chaque phase d'insufflation supplémentaire.

En procédant ainsi, on peut améliorer l'élasticité pulmonaire et/ou thoracique du patient grâce au volume gazeux cible insufflé dans ses poumons, voire provoquer un recrutement alvéolaire et/ou une mobilisation des sécrétions et l'expectoration.

Selon le cas, l'appareil à toux de l'invention peut comprendre l'une ou plusieurs des caractéristiques techniques suivantes :
- les moyens de pilotage sont configurés pour commander la turbine pour insuffler du gaz au patient pendant 2 à 10 phases d'insufflation initiales successives, de préférence pendant 2 à 5 phases d'insufflation initiales successives, de préférence encore pendant 3 phases d'insufflation initiales successives.
- à l'étape e), les moyens de pilotage commandent la turbine pour fournir un débit de gaz au patient, pendant au moins une phase d'insufflation supplémentaire.
- selon un mode de réalisation, les moyens de pilotage sont configurés pour fixer un volume de gaz cible (Vc) égal au volume de gaz moyen (Vm), c'est-à-dire tel que : Vc = Vm.
- selon un autre mode de réalisation, les moyens de pilotage sont configurés pour fixer un volume de gaz cible (Vc) supérieur au volume de gaz moyen (Vm), c'est-à-dire tel que : Vc > Vm.
- à l'étape e), la turbine fournit du gaz au patient pendant plusieurs phases d'insufflation supplémentaires suivant lesdites phases d'insufflation initiales.
- il comprend des moyens de réglage d'une durée de séance d'insufflation totale comprenant plusieurs phases d'insufflation supplémentaires, par exemple une durée de séance d'insufflation totale de 5 à 30 minutes, ou plus. Durant la séance d'insufflation, le patient reçoit du gaz.
- alternativement, la durée d'une séance d'insufflation est fixée par activation par l'utilisateur (i.e. patient ou aidant) de moyens de démarrage/arrêt de traitement, par exemple une ou des touches commandant un démarrage et un arrêt du traitement. Dans ce cas, la durée totale de la séance d'insufflation correspond au temps qui s'écoule entre le démarrage de la séance se produisant après appui sur une touche de démarrage de traitement et l'arrêt de la séance qui est commandé par un autre appui sur une touche d'arrêt de traitement, les touches de démarrage et d'arrêt de traitement pouvant être des touches différentes ou une même touche.
- à l'étape f), l'écran d'affichage est configuré pour afficher, pendant chaque phase d'insufflation supplémentaire, le volume de gaz insufflé (Vi) au patient, c'est-à-dire est une estimation de la quantité de gaz effectivement inhalée par le patient (elle ne tient pas compte des fuites éventuelles) et le volume cible (Vc).
- à l'étape f), l'écran d'affichage est configuré pour afficher une représentation graphique de volume de gaz comprenant au moins le volume de gaz insufflé (Vi) et le volume cible (Vc).
- à l'étape f), l'écran d'affichage est configuré pour afficher une représentation graphique comprenant un barre-graphe, ou analogue, par exemple un disque ou demi-disque, donnant au moins le volume de gaz insufflé (Vi) et le volume cible (Vc).
- à l'étape f), l'écran d'affichage est configuré pour afficher en outre une ou des valeurs numériques de volume de gaz insufflé (Vi) et/ou de volume cible (Vc).
- l'écran d'affichage est configuré pour afficher un barre-graphe évolutif représentatif de la réalisation de la phase d'insufflation en cours.
- la représentation graphique, en particulier le barre-graphe, affichée sur l'écran d'affichage comprend une échelle graduée représentative des volumes de gaz et un curseur mobile représentatif du volume insufflé, lors de la phase d'insufflation en cours.
- le barre-graphe comprend une échelle graduée représentative des volumes de gaz donnés en pourcentage (%) par rapport au volume cible (Vc).
- l'échelle graduée comprend des valeurs en pourcentage (%) par rapport au volume cible (Vc) comprises entre -15% et +15% (le volume cible Vc correspondant à la valeur de 0%).
- le curseur mobile se déplace par rapport à l'échelle graduée du barre-graphe ou analogue pour indiquer le volume de gaz insufflé par rapport au volume cible (Vc).
- le barre-graphe ou analogue est affiché en noir et blanc ou en couleurs.
- l'écran d'affichage est configuré pour afficher simultanément un barre-graphe à échelle graduée, un curseur mobile se déplaçant par rapport à l'échelle graduée du barre-graphe et des valeurs numériques de volume de gaz insufflé (Vi) et de volume cible (Vc).
- l'écran d'affichage est configuré pour continuer à afficher, pendant une phase d'exsufflation (i.e. d'expiration) suivant une phase d'insufflation, le volume de gaz insufflé (Vi) ayant été insufflé pendant ladite phase d'insufflation, de préférence pendant une phase d'exsufflation (i.e. d'expiration du patient), et une phase de pause suivant ladite phase d'insufflation. Autrement dit, on maintient un affichage du volume de gaz insufflé (Vi) déterminé en fin de phase d'insufflation (i.e. d'inspiration du patient), pendant la phase d'exsufflation et d'une éventuelle phase de pause.
- les moyens de pilotage sont configurés pour réinitialiser l'affichage du volume de gaz insufflé (Vi) au début de chaque phase d'insufflation supplémentaire, typiquement pour remettre à zéro, le volume de gaz insufflé (Vi).
- l'écran d'affichage est en outre configuré pour afficher un nombre de cycles d'insufflation réalisés.
- les moyens de pilotage sont configurés pour commander la turbine pour insuffler du gaz au patient pendant plusieurs phases d'insufflation successives à un débit donné jusqu'à obtenir une pression maximale préfixée ou une durée maximale d'inspiration donnée.
- la pression maximale, i.e. une pression de consigne, est comprise entre 10 et 50 mbar, par exemple de l'ordre de 30 à 40 mbar,
- la durée maximale d'inspiration donnée peut atteindre plusieurs secondes à une dizaines de secondes, voire plus, typiquement de l'ordre de 10 secondes ou moins.
- les moyens de pilotage sont en outre configurés pour afficher sur l'écran d'affichage, une animation vidéo mettant en oeuvre une représentation graphique symbolisant un patient pendant au moins une phase d'insufflation de gaz, et dans laquelle la forme, l'aspect et/ou les dimensions de ladite représentation graphique affichée sur l'écran d'affichage varient en corrélation avec le volume de gaz fourni par la turbine au patient, pendant ladite au moins une phase d'insufflation. Ceci permet d'aider et motiver le patient à maintenir son inspiration de gaz pendant toute la durée de la phase d'insufflation de gaz opérée par la turbine de l'appareil à toux de manière à ce qu'il inhale, si possible, tout le volume de gaz fourni. Ainsi, le patient n'a qu'à se baser sur la ou les variations de forme, d'aspect et/ou de dimensions de la représentation graphique affichée sur l'écran d'affichage de manière à continuer à inspirer tant que la représentation graphique varie.
- l'appareil à toux permet d'opérer plusieurs insufflations de gaz, chaque insufflation est suivie par une exsufflation de gaz, c'est-à-dire de sortie passive de gaz des poumons du patient en mode IPPB.
- l'animation vidéo met en oeuvre une représentation graphique symbolisant le patient pendant au moins une phase d'insufflation de gaz et au moins une phase d'exsufflation de gaz, ladite phase d'exsufflation de gaz étant consécutive à ladite phase d'insufflation de gaz.
- l'animation vidéo comprend une pluralité d'images successives.
- les moyens de pilotage sont configurés pour commander, en réponse à une activation par l'utilisateur d'une touche de démarrage d'insufflation ou d'inhalation, un démarrage de la turbine et simultanément de l'animation vidéo, c'est-à-dire un début de traitement du patient.
- alternativement, les moyens de pilotage sont configurés pour commander, en réponse à une détection d'une inspiration par l'utilisateur (i.e. début de phase inspiratoire avec inhalation de gaz), un démarrage de la turbine et simultanément de l'animation vidéo, c'est-à-dire un début de traitement du patient.
- il comprend des moyens de détection d'inspiration comprennent un capteur de pression, de préférence le capteur de pression est agencé dans l'appareil, par exemple au niveau de la sortie de gaz allant vers le patient.
- les moyens de détection d'inspiration coopèrent avec les moyens de pilotage pour leur fournir un signal représentatif d'un début de phase inspiratoire chez le patient.
- les moyens de pilotage sont configurés pour débuter le déroulé de l'animation vidéo affichée après activation par l'utilisateur des moyens de démarrage de traitement avec insufflation de gaz ou après détection d'un début de phase inspiratoire du patient.
- les moyens de démarrage/arrêt de traitement comprennent une touche de démarrage/arrêt unique (i.e. une seule et même touche) commandant le démarrage et l'arrêt d'un traitement.
- la (ou les) touche tactile est affichée sur l'écran d'affichage, c'est-à-dire que l'écran d'affichage est configuré pour afficher la (ou les) touche tactile.
- la (ou les) touche tactile coopère avec les moyens de pilotage.
- les variations de dimensions de la représentation graphique se font de manière progressive entre le début et la fin des phases d'insufflation et éventuellement d'exsufflation de gaz, c'est-à-dire des phases inspiratoires et/ou expiratoires du patient.
- les variations de dimensions de la représentation graphique se font de manière synchronisées avec une variation de couleur de la représentation graphique, en particulier une succession de couleurs, par exemple violet et vert, ou toute autre couleur(s).
- la représentation graphique représente ou comprend un animal ou un personnage, par exemple un caméléon ou tout autre animal, c'est-à-dire un animal ou un personnage symbolisant le patient qui suit le traitement par insufflation et exsufflation de gaz.
- la représentation graphique symbolise au moins une phase d'inspiration du patient, de préférence une phase d'inspiration et une phase d'expiration suivant ladite phase d'inspiration, et éventuellement une phase de pause (entre les phases d'inspiration et d'expiration, pendant laquelle le patient ni n'inspire, ni n'expire de gaz.
- les moyens de pilotage sont configurés pour commander la turbine de manière à insuffler du gaz au patient, typiquement de l'air.
- l'écran d'affichage est configuré pour afficher la vidéo d'animation en couleurs.
- l'appareil comprend en outre des moyens de mémorisation configurés pour mémoriser la pluralité d'images formant la vidéo d'animation, par exemple une mémoire informatique de type EEPROM ou analogue.
- l'affichage de la vidéo d'animation est opéré en temps réel pendant les phases d'insufflation et d'exsufflation de gaz.
- les moyens de sélection de mode de ventilation comprennent une ou des touches tactiles.
- l'appareil comprend en outre des moyens de sélection d'un mode de ventilation choisi parmi les modes INEX et IPPB.
- l'écran d'affichage est configuré pour afficher la ou les touches tactiles permettant de sélectionner le mode de ventilation choisi parmi les modes INEX et IPPB.
- le mode INEX correspond à une alternance de phases insufflatoire et exsufflatoire de gaz, éventuellement espacées par une phase de pause, avec insufflation de gaz à pression positive haute assez élevée, par exemple d'au moins 5 mbar, de préférence d'au moins 15 à 20 mbar, par exemple de l'ordre de 30 bar, pendant une durée d'insufflation courte, par exemple de 1 à 3 sec, suivi d'une exsufflation de gaz par mise en dépression (i.e. pression négative), par exemple de l'ordre de -30 mbar, des poumons et de la cage thoracique du patient pour provoquer la mobilisation et l'expulsion/extraction des sécrétions pulmonaires et/ou bronchiques La pression positive haute, la pression négative et les durées d'insufflation et/ou d'exsufflation sont réglables.
- le mode IPPB correspond à une alternance de phases insufflatoire et exsufflatoire de gaz, éventuellement espacées par une phase de pause, avec insufflation de gaz à débit faible, par exemple compris entre 5 et 100 L/min, par exemple entre 10 et 15 L/min, jusqu'à atteindre une pression de consigne maximale ou une durée d'insufflation maximale, typiquement une pression de consigne maximale entre 10 et 50 mbar, par exemple de l'ordre de 30 à 40 mbar, pendant une durée d'insufflation variable (pouvant atteindre de l'ordre de 10 secondes) et avec fourniture d'un volume de gaz variable, en fonction de l'élasticité des poumons et de la cage thoracique du patient, suivi d'une exsufflation de gaz sans mise en dépression (i.e. pression négative) des poumons et de la cage thoracique du patient, par exemple pendant une durée de 1 à 3 secondes, pour provoquer un recrutement alvéolaire et/ou une mobilisation des sécrétions et l'expectoration et/ou par ailleurs améliorer l'élasticité pulmonaire et/ou thoracique du patient grâce à la pression gazeuse appliquée à ses poumons. Le niveau de pression maximale (i.e. positive), le débit de gaz administré et/ou la durée d'insufflation maximale sont réglables.
- le déroulé de l'affichage de la vidéo d'animation est synchronisé avec le volume de gaz insufflé, lors des phases d'insufflation de gaz, en particulier en mode IPPB.
- il comprend en outre des moyens de réglage du débit de gaz (i.e. d'air) insufflé, de la pression maximale d'inspiration/d'insufflation (aussi appelée pression de consigne) et/ou de la durée maximale d'inspiration/d'insufflation, en mode IPPB, c'est-à-dire après sélection du mode IPPB.
- les moyens de pilotage sont configurés pour stopper, en réponse à une activation par l'utilisateur de moyens de démarrage/arrêt de traitement, la turbine et simultanément de l'animation vidéo, c'est-à-dire un arrêt de traitement du patient.
- la vidéo d'animation comprend plusieurs images différentes successives comprenant la représentation graphique dont la forme, l'aspect (y compris la couleur) et/ou les dimensions affichées sur l'écran d'affichage varient en corrélation avec les volumes de gaz lors des phases d'insufflation, en particulier en mode IPPB.
- l'écran d'affichage comprend en outre des moyens de sélection d'un sous-mode de fonctionnement, de préférence une (ou des) touche tactile affichée sur l'écran, permettant de sélectionner un sous-mode de fonctionnement choisi parmi les modes automatique (AUTO) et manuel. Le sous-mode de fonctionnement est sélectionnable uniquement après sélection préalable du mode de ventilation INEX.
- les moyens de pilotage sont configurés pour afficher sur l'écran d'affichage et démarrer l'animation vidéo uniquement après sélection par l'utilisateur du (sous-) mode automatique (AUTO), après sélection préalable du mode de ventilation IPPB.
- l'écran d'affichage est configuré pour afficher en outre, en mode IPPB, un volume de gaz courant, un volume de gaz cible, une durée de séance et/ou un nombre de cycle effectués.
- les moyens de pilotage sont configurés pour commander la turbine de manière à délivrer du gaz pendant les phases d'insufflation et éventuellement d'exsufflation de gaz au patient.
- il comprend une carcasse rigide.
- la turbine motorisée, la ligne d'insufflation, la ligne d'exsufflation et les moyens de pilotage sont agencés dans la carcasse.
- l'écran d'affichage est porté par la carcasse.
- l'écran d'affichage est fixé, i.e. solidarisé, de manière détachable ou non détachable à la carcasse.
- il comprend une interface homme-machine (IHM), aussi appelée interface graphique utilisateur (IGU), configurée pour permettre à un utilisateur d'opérer une ou des sélections ou des choix, de rentrer ou d'ajuster/modifier une ou des valeurs de consigne, notamment des valeurs de pression haute et basse, de temps des différentes phases...
- l'écran d'affichage fait partie de l'IHM.
- l'écran d'affichage est un écran numérique tactile, typiquement à affichage en couleurs.
- l'écran d'affichage comprend en outre une ou des touches tactiles permettant d'opérer des sélections, des validations, des réglages, des démarrages ou des arrêts de fonctionnement... Les touches tactiles sont à actionnement digital, c'est-à-dire qu'elles sont activées lorsque l'utilisateur appuie dessus avec son doigt, typiquement son index.
- l'écran d'affichage est configuré pour opérer un affichage d'informations sous forme de caractères alphanumériques, de représentations graphiques (e.g. graphes, courbes, dessins, icones, etc....), de photos, d'animations vidéo ou autres.
- le circuit de gaz comprend une ligne d'insufflation et une ligne d'exsufflation.
- la ligne d'insufflation est raccordée fluidiquement à la sortie de gaz de la turbine.
- la ligne d'exsufflation est accordée fluidiquement à l'entrée de gaz de la turbine.
- une ligne commune de fourniture de gaz à un patient raccordée fluidiquement auxdites ligne d'insufflation et ligne d'exsufflation.
- il comprend des valves pneumatiques agencées sur la ligne d'exsufflation et sur la ligne d'insufflation et des moyens de commande pneumatiques commandant pneumatiquement lesdites valves pneumatiques.
- les valves pneumatiques agencées sur la ligne d'exsufflation sont configurées pour contrôler une mise en communication fluidique de la ligne d'exsufflation avec l'atmosphère et/ou une mise en communication fluidique de la ligne commune de fourniture de gaz avec la ligne d'exsufflation.
- les valves pneumatiques agencées sur la ligne d'insufflation sont configurées pour contrôler une mise en communication fluidique de la ligne d'insufflation avec l'atmosphère et/ou une mise en communication fluidique de la ligne d'insufflation avec la ligne commune de fourniture de gaz.
- la turbine est configurée pour délivrer de l'air.
- une (chaque) phase d'insufflation a une durée comprise entre 0.5 et 20 secondes, typiquement jusqu'à 3 à 8 sec environ, par exemple de l'ordre de 6 sec.
- une (chaque) phase d'exsufflation a une durée comprise entre 0.5 et 10 secondes, typiquement jusqu'à 3 à 5 secondes environ.
- les moyens de pilotage sont configurés pour commander la turbine de manière à opérer, i.e. déclencher, cycliquement une (les) phase d'insufflation.
- de préférence, la (les) phase d'exsufflation est passive et suit une phase d'insufflation.
- optionnellement, la (chaque) phase d'insufflation et la (chaque) phase d'exsufflation qui la suit sont elles-mêmes suivies par une phase de pause, i.e. située entre une phase d'exsufflation et la phase d'insufflation suivante. De préférence, les durées des phases d'exsufflation et/ou de pause ne sont pas paramétables, i.e. non-réglables, en mode IPPB
- pendant la (chaque) phase de pause, la turbine est commandée par les moyens de pilotage pour délivrer une pression de pause supérieure ou égale à 0 mbar dans la ligne d'insufflation, de préférence une pression de pause entre 0 et 30 mbar, par exemple de l'ordre de 10 à 20 mbar.
- la (chaque) phase de pause a une durée qui dépend du patient et de son désir d'opérer une pause ou non, avant une nouvelle inspiration de gaz.
- les moyens de pilotage comprennent un (micro)contrôleur numérique relié électriquement à la turbine.
- les moyens de pilotage comprennent un (ou plusieurs) microprocesseur, de préférence porté par une carte électronique.
- les moyens de pilotage comprennent un (ou plusieurs) contrôleur numérique.
- le contrôleur numérique est relié électriquement à la turbine.
- le contrôleur numérique comprend une carte électronique à microcontrôleur mettant en oeuvre un ou plusieurs algorithmes.
- le contrôleur numérique comprend une carte électronique comprenant des moyens de mémorisation, telle une mémoire flash ou autre.
- la turbine comprend un moteur électrique, c'est-à-dire fonctionnant grâce à un courant électrique.
- la turbine comprend un moteur électrique entraînant une roue à ailettes agencée dans le compartiment interne d'une volute.
- pendant une phase d'insufflation et/ou une phase de pause éventuelle, la turbine délivre de l'air sous pression (i.e. > à la pression atmosphérique) dans la ligne d'insufflation raccordée fluidiquement à la sortie de gaz de la turbine, c'est-à-dire que la turbine génère une pression positive (P+) dans ladite ligne d'insufflation. L'air sous pression y chemine en direction de la ligne commune de fourniture de gaz et donc du patient.
- pendant une (chaque) phase d'insufflation en mode IPPB, la turbine génère une pression positive (P+) comprise entre 5 et 70 mbar (pression relative par rapport à la pression atmosphérique), de préférence une pression d'insufflation réglable inférieure ou égale à 50 mbar.
- pendant une (chaque) phase de pause éventuelle en mode IPPB, la turbine génère une pression positive (P+) comprise entre 0 et 30 mbar (pression relative par rapport à la pression atmosphérique) de préférence une pression d'insufflation réglable entre 1 et 20 mbar.
- pendant une (chaque) phase d'exsufflation en mode IPPB, le patient expire seul sans mise en dépression de ses poumons, c'est-à-dire naturellement.
- la turbine est commandée pour atteindre une vitesse de rotation maximale de 75 000 tours/minute, typiquement entre 10 000 et 50 000 tr/min.
- il comprend des moyens d'alimentation en courant électrique alimentant en courant électrique au moins la turbine motorisée, l'IHM et les moyens de pilotage et, éventuellement, la pompe ou le compresseur.
- les moyens d'alimentation en courant électrique comprennent au moins une batterie, de préférence rechargeable, et/ou une prise électrique et un cordon électrique de raccordement au secteur (e.g. 110/230 V), et éventuellement un transformateur de courant.
- le circuit de gaz, en particulier la ligne commune de fourniture de gaz, est relié fluidiquement au patient via une interface respiratoire, tel un masque respiratoire, par exemple un masque bucco-nasal, ou un embout buccal.
- la ligne commune de fourniture de gaz est reliée fluidiquement à l'interface respiratoire par l'intermédiaire d'un tuyau flexible ou analogue.

L'appareil à toux de l'invention est adapté au traitement des patients adultes, y compris les personnes âgées, mais aussi pédiatriques, c'est-à-dire les enfants ou les adolescents.

L'invention va maintenant être mieux comprise grâce à la description détaillée suivante, faite à titre illustratif mais non limitatif, en référence aux figures annexées parmi lesquelles :
- Fig. 1 est un schéma d'une architecture interne d'un appareil d'insufflation et d'exsufflation de gaz,
- Fig. 2 schématise un mode de réalisation de l'écran d'affichage d'un appareil à toux selon l'invention avant le démarrage d'un traitement, en mode IPPB, et
- Fig. 3 schématise l'écran d'affichage de Fig. 2 lors d'une insufflation de gaz en mode IPPB.

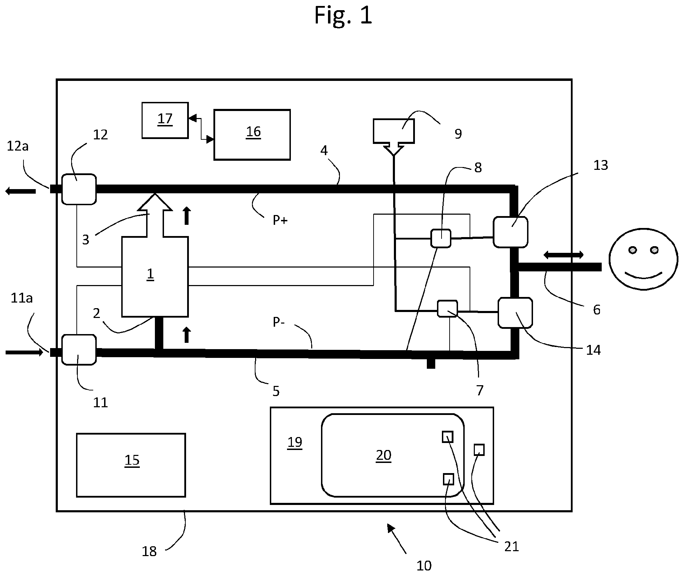

Fig. 1 schématise une architecture interne d'un appareil d'assistance à la toux 10 ou appareil à toux, tel celui de l'invention, permettant de réaliser des insufflations de gaz à un patient souffrant de troubles respiratoires nécessitant une aide à l'évacuation des sécrétions pulmonaires et/ou pour améliorer son élasticité pulmonaire et/ou thoracique, en particulier selon un mode IPPB. Cette architecture est classique et décrite en détail par EP-A-3622991 auquel on peut se reporter pour plus de détail.

Un tel appareil à toux peut aussi fonctionner selon un ou d'autres modes, notamment en mode INEX, dans lequel des insufflations et exsufflations de gaz successives sont opérées.

Schématiquement, l'appareil à toux 10 comprend une turbine 1 motorisée, aussi appelé compresseur ou (micro)soufflante, comprenant une entrée de gaz 2 par laquelle l'air est aspiré et pénètre dans la turbine 1 et une sortie de gaz 3 par laquelle l'air, de préférence à pression positive, est expulsé et ressort de la turbine 1. La turbine 1 comprend classiquement un moteur électrique entraînant une roue à ailettes agencée dans le compartiment interne de la volute et servant à délivrer de l'air sous pression (> 1 bar).

Il est également prévu des moyens de pilotage 16 commandant notamment la turbine 1, via une liaison électrique, pour pouvoir délivrer du gaz pendant des phases d'insufflation et éventuellement d'exsufflation, en fonction du mode choisi, notamment IPPB ou INEX.

Chaque séquence d'insufflation/d'exsufflation peut être suivie par une phase de pause si le patient le souhaite, située entre une phase d'exsufflation et la phase d'insufflation suivante. Pendant la phase de pause, la turbine 1 est commandée par les moyens de pilotage 16 pour délivrer une pression gazeuse comprise par exemple entre 0 et 20 mbar dans la ligne d'insufflation 4 et la ligne commune 6.

De préférence, les moyens de pilotage 16 comprennent un ou plusieurs microprocesseurs, typiquement un contrôleur numérique relié électriquement à la turbine 1. Le contrôleur numérique peut comprendre par exemple une carte électronique à microcontrôleur mettant en oeuvre un ou plusieurs algorithmes servant à piloter la turbine 1 et des moyens de mémorisation, telle une mémoire flash ou autre.

Pendant le fonctionnement de l'appareil 1, la turbine 1 alimente en air, une ligne d'insufflation 4 qui est raccordée fluidiquement à la sortie de gaz 3 de la turbine 1 et permet d'acheminer l'air expulsé par la turbine 1. Par ailleurs, une ligne d'exsufflation 5 est quant à elle raccordée fluidiquement à l'entrée de gaz 2 de la turbine 1 et permet notamment d'y acheminer l'air aspiré par la turbine 1. Les lignes d'insufflation 4 et d'exsufflation 5 sont par exemple des conduits de gaz, des passages de gaz ou analogues, agencés dans l'appareil à toux 10.

Les lignes d'insufflation 4 et d'exsufflation 5 sont par ailleurs raccordées fluidiquement à une ligne commune 6 de fourniture de gaz, tel un conduit de gaz ou analogue, qui est reliée fluidiquement à un patient, par exemple via un tuyau flexible relié fluidiquement à une interface respiratoire, tel qu'un masque respiratoire ou analogue, de sorte d'alimenter les voies respiratoires du patient, notamment ses poumons, en air sous pression pendant les phases d'insufflation et, à l'inverse, d'en extraire le gaz pendant les phases d'exsufflation et à l'aider ainsi à expulser ses sécrétions pulmonaires ou bronchiques.

Les lignes d'insufflation 4 et d'exsufflation 5 comprennent plusieurs valves pneumatiques 11, 12, 13, 14 permettant de contrôler les flux gazeux pendant les phases d'insufflation et d'exsufflation successives, et optionnellement pendant les phases de pause et/ou transitoires.

Schématiquement, une première 11 et une quatrième 14 valves pneumatiques sont agencées sur la ligne d'exsufflation 5. La première valve pneumatique 11 contrôle la mise en communication fluidique de la ligne d'exsufflation 5 avec l'atmosphère ambiante, via un orifice d'entrée 11a, pour permettre à de l'air atmosphérique de d'y pénétrer et d'alimenter la turbine 1, pendant les phases d'insufflation, alors que la quatrième valve pneumatique 14 contrôle la mise en communication fluidique de la ligne commune 6 de fourniture de gaz avec la ligne d'exsufflation 5, pendant les phases d'exsufflation.

Par ailleurs, une deuxième 12 et une troisième 13 valves pneumatiques sont agencées sur la ligne d'insufflation 4. La deuxième valve pneumatique 12 contrôle la mise en communication fluidique de la ligne d'insufflation 4 avec l'atmosphère, via un orifice d'évacuation de gaz 12a pour évacuer le gaz sous pression, pendant les phases d'exsufflation, comme expliqué ci-après, alors que la troisième valve pneumatique 13 contrôle la mise en communication fluidique de la ligne d'insufflation 4 avec la ligne commune 6 de fourniture de gaz, pour alimenter le patient en air, pendant les phases d'insufflation.

Selon le mode choisi, les phases d'exsufflation peuvent être actives (e.g. mode INEX) ou passives (e.g. en mode IPPB).

Ces valves pneumatiques 11-14 sont elles-mêmes pilotées pneumatiquement par des moyens de commande pneumatiques 7, 8 comprenant des première et seconde électrovannes pneumatiques 7, 8, à savoir des électrovannes miniatures à faible consommation de pic (i.e. < 10 Watt) qui sont pilotées électriquement, via une ou plusieurs liaisons électriques, par les moyens de pilotage 16 de l'appareil à toux 10, en fonction de la phase d'insufflation ou d'exsufflation à opérer, et/ou des éventuelles phases de pause.

Les première et seconde électrovannes pneumatiques 7, 8 sont par ailleurs reliées pneumatiquement à au moins une ligne de pilotage pneumatique, c'est-à-dire au moins une ligne d'amenée de pression positive, par exemple une conduite de gaz qui se ramifie ou plusieurs conduites, reliant ces première et seconde électrovannes pneumatiques 7, 8 à une source de pression positive 9, i.e. une source de gaz sous pression, tel qu'une micro-pompe ou micro-compresseur additionnel.

En outre, les première et seconde électrovannes 7, 8 sont aussi connectées fluidiquement à une source de pression dite « négative », appelée simplement source de pression négative, via au moins une ligne d'amenée de pression négative, par exemple à la ligne d'exsufflation 5 où règne une pression négative (P-) pendant les phases d'exsufflation.

Le fonctionnement et la structure de ces valves pneumatiques et électrovannes sont détaillés dans EP-A-3622991, auquel on peut se reporter pour plus de détail.

Comme déjà dit, un tel appareil peut être utilisé pour mettre en oeuvre des modes INEX, IPPB ou autres ; toutefois, seul le mode IPPB est décrit en détail ci-après.

Ainsi, en mode IPPB, l'insufflation de gaz par la turbine 1 s'effectue à débit faible, par exemple de l'ordre de 5 à 100 mL/Min, pendant une durée d'insufflation réglable pouvant atteindre environ 20 sec, par exemple de l'ordre de 6 sec, pendant laquelle du gaz est insufflé jusqu'à atteindre une pression de consigne donnée ou la fin de la durée d'insufflation préfixée, pour faire varier, i.e. augmenter, progressivement et de manière douce l'élasticité de la cage thoracique et des poumons du patient.

Chaque phase d'insufflation est suivie d'une phase exsufflation ou sortie de gaz des poumons du patient jusqu'à ce que la cage thoracique et les poumons du patient reviennent à leur état de repos et ce, de manière passive, c'est-à-dire sans avoir recours à une mise en dépression, en mode IPPB.

Autrement dit, pendant une phase d'insufflation, la turbine 1 génère une pression positive (P+) du côté de la sortie 3 de gaz de la turbine 1, c'est-à-dire une surpression, dans la ligne d'insufflation 4 et dans la ligne commune 6 de fourniture de gaz qui est fluidiquement raccordée à la ligne d'insufflation 5, pendant une durée d'insufflation assez longue, i.e. jusqu'à environ 20 secondes, du fait du débit de gaz faible appliqué jusqu'à atteindre pression de consigne maximale ou une durée maximale.

Ensuite, pendant la phase d'exsufflation qui suit la phase d'insufflation, le gaz expiré par le patient chemine successivement dans la ligne commune 6 et dans la ligne d'exsufflation 5, ce qui permet d'évacuer naturellement le gaz contenu dans le tractus respiratoire du patient, en particulier dans ses poumons, en engendrant un retour naturel de la cage thoracique et des poumons du patient à leur état de repos, c'est-à-dire que les poumons se vident tout seul sans assistance de la turbine, en mode IPPB.

Le gaz expiré est ensuite évacué vers l'atmosphère ambiante, au travers de la turbine 1, via l'orifice de mise à l'atmosphère 12a de la deuxième valve pneumatique 12.

Le débit de gaz, la durée maximale d'inspiration et la pression maximale d'inspiration, c'est-à-dire la pression de consigne maximale, sont réglables en mode IPPB.

Pendant les phases d'insufflation, la turbine 1 est commandée par les moyens de pilotage 16. La vitesse de rotation maximale de la turbine peut atteindre environ 75 000 tours/minute, typiquement entre 10 000 et 50 000 tr/min.

Les éléments de l'appareil à toux 10 montrés sur la Fig. 1 peuvent être agencés dans une carcasse 18 ou coque externe rigide, par exemple une carcasse en polymère ou analogue.

Une interface homme-machine 19 ou IHM, agencée sur la carcasse 18, permet à l'utilisateur de rentrer des valeurs de consigne dans l'appareil à toux 10, d'opérer des sélections ou des choix... L'IHM 19 comprend un écran tactile 20, de préférence à affichage en couleurs, et des touches 21 de sélection ou autres, notamment numériques tactiles s'affichant sur l'écran tactile 20, i.e. écran digital.

Des moyens d'alimentation 15 en courant électrique (non montrés) alimentent en courant électrique les différents composants de l'appareil à toux 10 nécessitant de l'être, à savoir notamment la turbine 1 motorisée, les moyens de pilotage 16, l'IHM 19, l'écran d'affichage 20... Les moyens d'alimentation 15 en courant électrique comprennent par exemple une (ou plusieurs) batterie, de préférence rechargeable, et/ou une prise électrique et un cordon électrique (non montré) de raccordement au secteur, e.g. 110/230 V, avec ou sans transformateur de courant.

D'une manière générale, un appareil à toux 10 est destiné à être utilisé pour opérer des insufflations de gaz (i.e. d'air) à des patients incapables de gérer seuls leurs sécrétions et/ou pour améliorer leur élasticité pulmonaire et thoracique, que ces patients soient des patients adultes ou pédiatriques.

L'appareil à toux 10 est préférentiellement suffisamment léger et compact pour être facilement transportable. Il peut être utilisé à domicile ou à l'hôpital, avec des interfaces respiratoires invasives (e.g. sondes trachéales) ou non-invasives (e.g. masques).

Les moyens de pilotage 16 sont aussi configurés pour commander l'affichage d'informations de toute nature, à savoir des caractères alphanumériques, des icônes, des représentations graphiques, des courbes ou autres, sur l'écran d'affichage 20 de l'IHM 19, lequel opère alors un affichage de ces informations de manière à assister, informer ou autre, l'utilisateur.

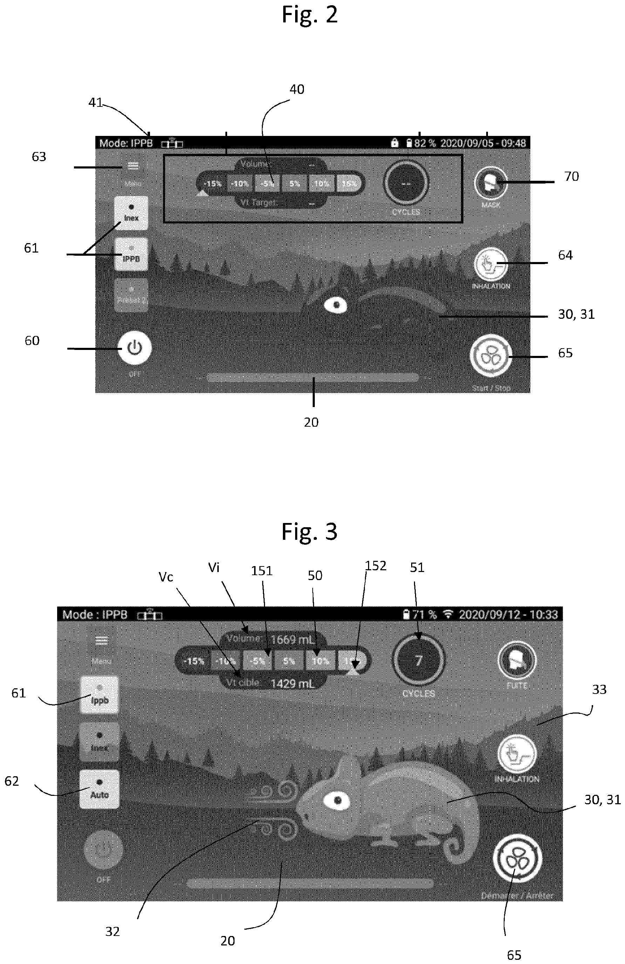

Afin de permettre l'utilisation de l'appareil à toux 10 par une personne moins formée qu'un kinésithérapeute, par exemple une auxiliaire de vie, une personne de l'entourage familial du patient... afin de pouvoir déclencher chez le patient, une séance d'utilisation de l'appareil à toux 10, au moment le plus opportun pour le patient considéré, sans avoir à faire venir spécialement un professionnel de santé qualifié de type kinésithérapeute ou analogue, et à attendre son passage, les moyens de pilotage 16 peuvent être configurés pour commander un affichage simultané, sur l'écran d'affichage 20 tactile, une animation vidéo mettant en oeuvre une représentation graphique 30 symbolisant le patient qui doit être traité, et dans laquelle la forme, l'aspect et/ou les dimensions de cette représentation graphique 30 affichée sur l'écran d'affichage 20 varient en corrélation avec les phases d'insufflation par la turbine 2 de l'appareil à toux 10, et préférentiellement d'exsufflation de gaz, comme illustré sur les Fig. 2 et Fig. 3.

Dans le mode de réalisation illustré sur ces Fig. 2 et Fig. 3, concernant le mode IPPB, la représentation graphique 30 symbolisant le patient, en particulier les phases inspiratoires dudit patient pendant le traitement, est représentée par un animal, à savoir ici un caméléon 31. Bien entendu, tout autre contenant et/ou autre animal ou personnage peuvent convenir.

Plus précisément, afin d'assister une personne n'ayant pas ou peu de connaissances médicales, par exemple une auxiliaire de vie ou une personne de l'entourage familial du patient (les aidants), dans la mise en oeuvre d'un appareil à toux 10 selon l'invention de manière à opérer au moins des insufflations de gaz à un patient souffrant de troubles respiratoires, les moyens de pilotage 16 commandent l'affichage sur l'écran d'affichage 20, dans le mode de réalisation proposé en exemple, à savoir ici en mode IPPB, de la vidéo d'animation mettant en scène ici un animal, à savoir ici un caméléon 31, en tant que représentation graphique 30 symbolisant le patient et ses phases respiratoires (i.e. inspiratoire et expiratoire), et préférentiellement une phase de pause optionnelle.

Dans cette animation vidéo, une représentation d'un flux de gaz 32 symbolise le débit de gaz insufflé au caméléon 31. Bien entendu, des représentations graphiques additionnelles ou différentes sont possibles.

On voit aussi que l'écran 20 comprend un fond d'écran 33 comprenant un décor ou analogue, à savoir ici une représentation de paysage de montagnes. Le caméléon 31 et la ou les représentations graphiques additionnelles 32, viennent se superposer audit paysage afin de créer une ambiance agréable et ludique pour l'utilisateur.

L'appareil à toux 10 comprend par ailleurs des moyens de mémorisation 17, telle qu'une carte mémoire, par exemple une EEPROM ou directement dans un exécutable (i.e. programme de processeur) ou autre, pour mémoriser la vidéo d'animation (i.e. animation vidéo).

Les moyens de pilotage 16 sont donc configurés pour aller retrouver dans les moyens de mémorisation 17 et ensuite afficher à l'écran 20, l'animation vidéo au début d'un traitement, en particulier au démarrage d'une phase d'insufflation de gaz, après sélection du mode de ventilation désiré, à savoir ici le mode IPPB, et ensuite activation (i.e. appui digital) par l'utilisateur d'une touche tactile 65 de démarrage/arrêt de traitement, comme détaillé ci-après. La touche 65 permet aussi, lorsqu'elle est activée pendant le déroulé d'un traitement, d'arrêter un traitement, c'est-à-dire l'envoi d'un débit de gaz au patient.

Après appui sur la touche tactile 65 de démarrage/arrêt de traitement, l'appareil se met en pause jusqu'au début d'une phase inspiratoire du patient qui peut être détectée par des moyens de détection d'inspiration comme expliqué ci-après, ou alors initiée par appui du patient ou d'un aidant sur la touche tactile 64 de démarrage de cycle servant à déclencher manuellement une phase d'insufflation par l'appareil 1.

En effet, l'appareil comprend des moyens de détection d'inspiration, tel un système déclencheur (« trigger » en anglais), configurés pour opérer un démarrage de l'insufflation de gaz, après détection d'une inspiration du patient, c'est-à-dire d'un début de phase inspiratoire, en utilisant par exemple un capteur de pression qui est agencé au niveau de la sortie patient et qui fournit des mesures de pression aux moyens de pilotage, typiquement un microprocesseur, qui les analysent pour en déduire le début d'une phase inspiratoire initiée par le patient.

Autrement dit, en mode IPPB :
- le démarrage de l'insufflation, selon le mode de réalisation, soit par déclenchement manuel, par exemple après appui sur la touche tactile 64, soit à l'aide de moyens de détection d'inspiration, tel un système déclencheur respiratoire, détectant un début d'inspiration de la part du patient lui-même, de préférence la sensibilité du système déclencheur est réglable.
- la phase d'inspiration se fait avec un débit réglable et se termine soit quand la pression maximale préfixée est atteinte, soit quand la durée maximale d'inspiration préréglée est atteinte.
- la phase d'expiration est passive afin que le patient puisse relâcher tout le gaz emmagasiné dans ses poumons. Eventuellement, un "soutien expiratoire" est mis en place, c'est à dire une légère résistance à l'expiration pour allonger le temps d'expiration du patient.
- la durée totale d'une séance de traitement peut être éventuellement réglée, comme expliqué ci-après.

On voit par ailleurs sur Fig. 2, qui montre l'écran 20 avant le démarrage d'un traitement, que l'écran d'affichage 20 de l'IHM 19 comprend d'autres éléments, tels des moyens d'activation, de sélection ou analogue, en particulier des touches de préréglage permettent de sélectionner/activer, i.e. d'utiliser, des préréglages (appelées 'presets' en anglais) préenregistrés, i.e. mémorisés, c'est-à-dire des réglages particuliers (pression, durées, pause, etc...) adaptés à certaines situations cliniques données, par exemple pour la nuit, pour le matin...

Par exemple, l'écran d'affichage 20 de l'IHM 19 comprend dans le mode de réalisation proposé :
- une touche tactile 60 de mise en route ou arrêt (OFF) de l'appareil.
- des touches tactiles 61 pour régler ou sélectionner des préréglages adaptés aux modes de ventilation INEX et IPPB, par exemple pour régler la durée ou temps maximal d'insufflation et/ou la pression de consigne maximale en mode IPPB.
- une touche tactile 62 pour sélectionner un préréglage (*preset*) pour le sous-mode de fonctionnement automatique (AUTO).
- une touche tactile 65 de démarrage/arrêt de traitement pour démarrer et/ou stopper le fonctionnement de la turbine 2.
- une touche tactile 63 pour accéder à différents menus ou sélections.
- une touche tactile 64 pour déclencher manuellement une inspiration, c'est-à-dire pour commencer une insufflation de gaz.
- un indicateur visuel coloré 70 de bon positionnement du masque. Si le masque est mal positionné et que l'appareil détecte une fuite trop importante, l'indicateur 70 s'allume à la fin de l'inspiration, par exemple en orange ou en une autre couleur.

Selon l'invention, les moyens de pilotage 16, notamment un (ou des) microprocesseur mettant en oeuvre un (ou des) algorithmes, sont configurés pour commander la turbine pour insuffler du gaz au patient, i.e. un débit gazeux, pendant plusieurs phases d'insufflation initiales successives et déterminer les volumes de gaz insufflés Vi au patient pendant ces phases d'insufflation initiales successives, par exemple pendant 3 à 5 phases d'insufflation initiales.

Chaque phase d'insufflation est suivie d'une phase d'exsufflation (i.e. expiration) de gaz par le patient, c'est-à-dire de sortie de gaz enrichi en CO₂ des poumons du patient. Comme déjà dit, en mode IPPB, cette exsufflation ou sortie de gaz se fait passivement.

Le début ou démarrage de chaque phase d'insufflation est opéré, comme déjà expliqué, soit par déclenchement manuel, par exemple après appui sur la touche tactile 64, soit à l'aide de moyens de détection d'inspiration, tel un système déclencheur respiratoire, détectant un début d'inspiration de la part du patient lui-même. Une phase d'insufflation peut donc être séparée de la phase d'insufflation suivante de plusieurs secondes à une ou plusieurs dizaines de secondes, notamment en fonction du patient à traiter.

Après démarrage d'une phase d'insufflation de gaz, le gaz est fourni par la turbine au patient à un débit donné, de préférence un débit réglable, et se termine quand une pression maximale préfixée est atteinte ou quand une durée maximale d'inspiration préréglée est atteinte.

Les volumes (e.g. V₁, V₂... Vᵢ) de gaz insufflés successivement au patient pendant ces i phases d'insufflation initiales successives, avec typiquement i > 2, par exemple i compris entre 3 et 5, servent à calculer un volume de gaz moyen Vm, par exemple en appliquant la formule suivante : Vm = (V₁ + V₂ + ... + Vi) / i

Ensuite, les moyens de pilotage 16 servent à déterminer un volume de gaz cible Vc à partir du volume de gaz moyen Vm, tel que : Vc ≥ Vm, c'est-à-dire que le volume de gaz cible Vc peut être égal ou supérieur au volume de gaz moyen Vm.

A titre d'exemple, on peut décider que le volume de gaz cible Vc est égal au volume de gaz moyen Vm.

Les volumes (e.g. V₁, V₂... Vᵢ) de gaz insufflés, le volume moyen Vm et/ou le volume de gaz cible Vc peuvent être mémorisés par des moyens de mémorisation, telle une mémoire informatique ou analogue, de préférence temporairement jusqu'au prochain démarrage d'un nouveau traitement où ces valeurs sont réinitialisées.

Puis, les moyens de pilotage 16 commandent la turbine 1 pour fournir du gaz au patient, i.e. un débit gazeux, pendant une ou plusieurs phases d'insufflation supplémentaires suivant lesdites phases d'insufflation initiales. Le début de chaque phase d'insufflation supplémentaire est initié par déclenchement manuel ou à l'aide de moyens de détection d'inspiration, comme précédemment.

Durant chaque phase d'insufflation supplémentaire le patient va tenter d'inhaler la quantité de gaz permettant d'arriver jusqu'au volume cible Vc, ce qui permet améliorer progressivement l'élasticité pulmonaire et thoracique du patient en « forçant » ses poumons à recevoir et contenir une quantité de gaz (i.e. Vc) supérieure ou égale à la quantité moyenne (Vm) correspondant aux premières insufflations.

Afin d'aider le patient, selon l'invention, on opère un affichage sur l'écran d'affichage 20 des volumes de gaz insufflé et cible, c'est-à-dire qu'on affichage un suivi en direct du volume de gaz qu'il inhale pendant le déroulé de chaque phase insufflatoire.

Plus précisément, comme visible sur Fig. 3, les moyens de pilotage commandent l'écran d'affichage 20 pour opérer un affichage, par exemple au sein d'une représentation graphique 40 située en haut d'écran 20, du volume d'air insufflé Vi (i.e. valeur instantanée au cours de la phase d'inspiration en cours), du volume cible Vc de la séance (e.g. déterminé sur la base de la moyenne des premières inspirations de la séance) et éventuellement du nombre de cycles de respiration 51 effectués depuis le début de la séance de traitement du patient.

Ici, la représentation graphique 40 inclut un barre-graphe 50 donnant une information de réalisation de l'insufflation du volume cible Vc, à savoir ici un pourcentage (i.e. %) de l'accomplissement de l'insufflation du volume cible Vc, à savoir ici entre -15% et +15%. D'autres représentations graphiques sont possibles.

Le barre-graphe 50 comprend une échelle graduée 151 représentative des volumes de gaz (en %) et un curseur mobile 152 représentatif du volume insufflé, pendant chaque une phase d'insufflation. Le curseur mobile 152 se déplace face à l'échelle graduée 151 pour indiquer le volume de gaz insufflé Vi (entre -15% et +15%) par rapport au volume cible Vc et ainsi aider visuellement le patient pendant sa thérapie en lui fournissant une information sur la progression de son inhalation de gaz.

Par ailleurs, l'écran d'affichage 20 comprend aussi, dans le mode de réalisation proposé, un bandeau haut 41, dans lequel sont affichées différentes informations utiles comme le rappel du mode, comme ici le mode IPPB par exemple, ayant été sélectionné, une icône d'autonomie de la batterie électrique (ici 82%), une icône de transmission sans fil, par exemple en wifi, la date, l'heure.... ou autres.

Fig. 3 schématise l'affichage sur l'écran d'affichage 20 lors d'une insufflation de gaz montrant aussi l'animation vidéo 30,31 mise en oeuvre par les moyens de pilotage 16 comprenant le caméléon 31. Sur la Fig. 2, le caméléon 31 est représenté (i.e. forme/aspect) totalement dégonflé, donc avec des dimensions minimales, alors qu'à l'inverse, le caméléon 41 est représenté gonflé de gaz sur la Fig. 3.

Au début d'une phase d'insufflation de gaz, i.e. d'air, les moyens de pilotage 16 commandent l'affichage sur l'écran 20 de l'animation vidéo 30, 31, après appui par l'utilisateur sur une touche tactile 64 de démarrage d'inhalation, de manière à montrer le caméléon 31 se gonflant progressivement, c'est-à-dire que ses dimensions et son aspect/sa forme augmentent à l'écran 20, mimant ainsi une entrée progressive de gaz (i.e. comme le font les poumons du patient).

Préférentiellement, lors d'une phase d'exsufflation de gaz, l'animation vidéo 30, 31 montre l'inverse, à savoir le caméléon 31, i.e. une représentation graphique 30 symbolisant le patient pendant son traitement, qui se dégonfle mimant ainsi une sortie de gaz des poumons du patient.

Le déroulé de l'affichage de la vidéo d'animation 30, 31 est synchronisé avec les volumes de gaz, lors des phases d'insufflation de gaz en mode IPPB.

Le flux gazeux 32 va vers le caméléon 31 (cf. Fig. 3) pour schématiser la circulation du gaz en direction du patient ou dans le sens opposé pour schématiser la sortie de gaz des poumons du patient. Sur la Fig. 2, l'absence d'affichage de flux gazeux 32 montre que le traitement n'a pas encore débuté.

Pendant une éventuelle phase de pause (non montrée) suivant une phase d'exsufflation de gaz, l'animation vidéo montre un caméléon 31 non-évolutif, c'est-à-dire que avec forme, dimensions et aspect qui ne changent pas à l'écran 20 étant donné qu'aucun échange gazeux ne se produit entre eux.

Autrement dit, de façon schématique, l'écran 20 affiche une animation vidéo qui montre:
- pendant chaque inspiration/insufflation (cf. Fig. 3), un caméléon 31 (i.e. patient) qui gonfle et change de couleur, passant par exemple du violet au vert en fonction du % du volume de gaz insufflé au patient
- pendant chaque expiration/l'exsufflation, un caméléon 31 (i.e. patient) qui dégonfle, et
- pendant chaque pause éventuelle, un caméléon 31 qui ne varie pas.

En affichant de telles représentations sur l'écran 20, l'appareil à toux 10 procure une aide visuelle très utile au patient et à la personne qui l'assiste lors de la mise en oeuvre de son traitement. Ainsi, le caméléon varie (dimensions, couleurs...) en fonction de l'effort fourni pour le patient qui inspire le gaz. L'animation encourage donc le patient à gonfler le plus possible ses poumons en inhalant si possible tout le volume gazeux qui lui est apporté pendant chaque phase inspiratoire.

L'animation vidéo 30, 31 et le barre-graphe 50 comprenant l'échelle graduée 151 représentative des volumes de gaz, notamment Vc, et le curseur mobile 152 représentatif du volume insufflé Vi, affichés sur l'écran d'affichage 20 pendant chaque une phase d'insufflation constituent une véritable aide au traitement. En effet, en les regardant, notamment en calquant sa respiration sur ces affichages, le patient est assuré de bien réaliser son traitement, c'est-à-dire d'obtenir un traitement efficace, en particulier pour améliorer son élasticité pulmonaire et/ou thoracique, et ce, sans nécessiter la présence sur place d'un personnel soignant qualifié, de type kinésithérapeute ou analogue.

Lorsque le patient a bien réalisé son traitement, c'est-à-dire à bien calqué sa respiration sur les phases d'insufflations de l'appareil à toux, les moyens de pilotage 16 peuvent être en outre aussi configurés pour afficher sur l'écran d'affichage 20, une animation vidéo additionnelle symbolisant cette bonne réalisation de son traitement par le patient, par exemple une animation vidéo additionnelle du type pluie de confetti ou une récompense comme une coupe ou un trophée, ou autre.

L'appareil d'assistance à la toux ou appareil à toux selon l'invention permet d'opérer efficacement des insufflations de gaz à un patient souffrant de troubles respiratoires nécessitant une aide à l'évacuation des sécrétions pulmonaires et/ou pour améliorer son élasticité pulmonaire et/ou thoracique, même lorsque le patient est assisté par une personne n'ayant pas ou peu de connaissances médicales, par exemple une auxiliaire de vie ou une personne de l'entourage familial du patient, encore appelés un « aidant », puisqu'il lui suffit de calquer sa respiration sur l'animation vidéo qui s'affiche à l'écran.

## Revendications

1. Appareil à toux (10) pour opérer au moins une insufflation de gaz à un patient, comprenant :
- une turbine motorisée (1) en communication fluidique avec un circuit de gaz (4, 5, 6) comprenant une ligne d'insufflation (4),
- un écran d'affichage (20), et
- des moyens de pilotage (16) configurés pour commander la turbine (1) et/ou un affichage sur l'écran d'affichage (20),
**caractérisé en ce que** les moyens de pilotage (16) sont en outre configurés pour:
a) commander la turbine (1) pour insuffler du gaz au patient pendant plusieurs phases d'insufflation initiales successives,
b) déterminer les volumes de gaz insufflés (Vi) au patient pendant lesdites plusieurs phases d'insufflation initiales successives,
c) déterminer au moins un volume de gaz moyen (Vm) à partir des volumes de gaz insufflés (Vi) pendant lesdites plusieurs phases d'insufflation initiales successives,
d) déterminer ou fixer un volume de gaz cible (Vc) à administrer au patient à partir du volume de gaz moyen (Vm), tel que : Vc ≥ Vm,
e) après avoir déterminé ou fixé le volume de gaz cible (Vc), commander la turbine (1) pour fournir du gaz au patient, pendant au moins une phase d'insufflation supplémentaire suivant lesdites phases d'insufflation initiales, et
f) commander un affichage sur l'écran d'affichage (20), pendant chaque phase d'insufflation supplémentaire, d'au moins le volume de gaz cible (Vc) et le volume de gaz insufflé (Vi) administré au patient pendant chaque phase d'insufflation supplémentaire.

2. Appareil selon la revendication 1, **caractérisé en ce que** les moyens de pilotage (16) sont configurés pour commander la turbine (1) pour insuffler du gaz au patient pendant 2 à 10 phases d'insufflation initiales successives.

3. Appareil selon la revendication 1, **caractérisé en ce qu'**à l'étape f), l'écran d'affichage (20) est configuré pour afficher une représentation graphique (40) de volume de gaz comprenant au moins le volume de gaz insufflé (Vi) et le volume cible (Vc).

4. Appareil selon la revendication 3, **caractérisé en ce que** la représentation graphique (40) comprend un barre-graphe (50) donnant au moins le volume de gaz insufflé (Vi) et le volume cible (Vc).

5. Appareil selon la revendication 1, **caractérisé en ce qu'**à l'étape f), l'écran d'affichage (20) est en outre configuré pour afficher une ou des valeurs numériques de volume de gaz insufflé (Vi) et/ou de volume cible (Vc).

6. Appareil selon l'une des revendications 3 ou 4, **caractérisé en ce que** la représentation graphique (40), en particulier le barre-graphe (50), affichée sur l'écran d'affichage (20) comprend une échelle graduée (151) représentative des volumes de gaz et un curseur mobile (152) représentatif du volume insufflé, pendant chaque une phase d'insufflation.

7. Appareil selon la revendication 1, **caractérisé en ce que** l'écran d'affichage (20) est configuré pour continuer à afficher, pendant une phase d'exsufflation suivant une phase d'insufflation du patient, le volume de gaz insufflé (Vi) ayant été insufflé pendant ladite phase d'insufflation, de préférence pendant une phase d'exsufflation et une phase de pause suivant ladite phase d'insufflation.

8. Appareil selon la revendication 1, **caractérisé en ce que** les moyens de pilotage (16) sont configurés pour réinitialiser l'affichage du volume de gaz insufflé (Vi) au début de chaque phase d'insufflation supplémentaire

9. Appareil selon la revendication 1, **caractérisé en ce que** l'écran d'affichage (20) est en outre configuré pour afficher en outre un nombre (51) de cycles d'insufflation réalisés.

10. Appareil selon la revendication 1, **caractérisé en ce que** l'écran d'affichage (20) est en outre configuré pour afficher en outre une animation vidéo (30, 31) mettant en oeuvre une représentation graphique (30) symbolisant un patient (31) pendant au moins une phase d'insufflation de gaz, et dans laquelle la forme, l'aspect et/ou les dimensions de ladite représentation graphique (30, 31) varient en corrélation avec le volume de gaz fourni par la turbine (2) au patient, pendant ladite au moins une phase d'insufflation.

11. Appareil selon la revendication 1, **caractérisé en ce que** les moyens de pilotage (16) sont configurés pour commander la turbine (1) pour insuffler du gaz au patient pendant plusieurs phases d'insufflation initiales successives à un débit donné jusqu'à obtenir une pression maximale préfixée ou une durée maximale d'inspiration donnée.

12. Appareil selon la revendication 1, **caractérisé en ce qu'**il comprend des moyens de réglage d'une durée de séance d'insufflation totale comprenant plusieurs phases d'insufflation supplémentaires, de préférence une durée de séance d'insufflation totale de 5 à 30 minutes.

13. Appareil selon l'une des revendications 4 ou 6, **caractérisé en ce que** le barre-graphe (50) comprend une échelle graduée (151) représentative des volumes de gaz donnés en pourcentage (%) par rapport au volume cible (Vc), de préférence des valeurs en pourcentage (%) comprises entre -15% et +15%, le volume cible Vc correspondant à la valeur de 0%.

14. Appareil selon l'une des revendications 6 ou 13, **caractérisé en ce que** le curseur mobile (152) se déplace par rapport à l'échelle graduée (151) du barre-graphe (50) pour indiquer le volume de gaz insufflé par rapport au volume cible (Vc).

15. Appareil selon la revendication 1, **caractérisé en ce que** les moyens de pilotage (16) sont configurés pour débuter le déroulé de l'animation vidéo affichée après activation par l'utilisateur de moyens de démarrage de traitement avec insufflation de gaz ou après détection d'un début de phase inspiratoire du patient par des moyens de détection d'inspiration.

## Patentansprüche

1. Hustengerät (10) zum Vornehmen mindestens einer Insufflation von Gas bei einem Patienten, umfassend:
- eine motorbetriebene Turbine (1) in Fluidverbindung mit einem Gaskreislauf (4, 5, 6), umfassend eine Insufflationsleitung (4),
- einen Anzeigebildschirm (20) und
- Ansteuerungsmittel (16), die dazu ausgestaltet sind, die Turbine (1) und/oder eine Anzeige auf dem Anzeigebildschirm (20) zu steuern,
**dadurch gekennzeichnet, dass** die Ansteuerungsmittel (16) ferner dazu ausgestaltet sind:
a) die Turbine (1) zu steuern, um bei dem Patienten Gas während mehrerer aufeinander folgender anfänglicher Insufflationsphasen zu insufflieren,
b) die insufflierten Gasvolumina (Vi) zu bestimmen, die bei dem Patienten während der mehreren aufeinander folgenden anfänglichen Insufflationsphasen insuffliert werden,
c) mindestens ein durchschnittliches Gasvolumen (Vm) ausgehend von den während der mehreren aufeinander folgenden anfänglichen Insufflationsphasen insufflierten Gasvolumina (Vi) zu bestimmen,
d) ein dem Patienten zu verabreichendes Zielgasvolumen (Vc) ausgehend von dem durchschnittlichen Gasvolumen (Vm) zu bestimmen oder festzulegen, so dass: Vc ≥ Vm,
e) nachdem das Zielgasvolumen (Vc) bestimmt oder festgelegt wurde, die Turbine (1) zu steuern, um Gas an den Patienten während mindestens einer zusätzlichen Insufflationsphase nach den anfänglichen Insufflationsphasen abzugeben, und
f) ein Anzeigen auf dem Anzeigebildschirm (20), während jeder zusätzlichen Insufflationsphase, mindestens des Zielgasvolumens (Vc) und des insufflierten Gasvolumens (Vi), das dem Patienten während jeder zusätzlichen Insufflationsphase verabreicht wird, zu steuern.

2. Gerät nach Anspruch 1, **dadurch gekennzeichnet, dass** die Ansteuerungsmittel (16) dazu ausgestaltet sind, die Turbine (1) zu steuern, um bei dem Patienten während 2 bis 10 aufeinander folgenden anfänglichen Insufflationsphasen Gas zu insufflieren.

3. Gerät nach Anspruch 1, **dadurch gekennzeichnet, dass** im Schritt f) der Anzeigebildschirm (20) dazu ausgestaltet ist, eine grafische Darstellung (40) des Gasvolumens anzuzeigen, die mindestens das insufflierte Gasvolumen (Vi) und das Zielvolumen (Vc) umfasst.

4. Gerät nach Anspruch 3, **dadurch gekennzeichnet, dass** die grafische Darstellung (40) ein Balkendiagramm (50) umfasst, das mindestens das insufflierte Gasvolumen (Vi) und das Zielvolumen (Vc) angibt.

5. Gerät nach Anspruch 1, **dadurch gekennzeichnet, dass** im Schritt f) der Anzeigebildschirm (20) ferner dazu ausgestaltet ist, einen oder mehrere Zahlenwerte für das insufflierte Gasvolumen (Vi) und/oder das Zielvolumen (Vc) anzuzeigen.

6. Gerät nach einem der Ansprüche 3 oder 4, **dadurch gekennzeichnet, dass** die auf dem Anzeigebildschirm (20) angezeigte grafische Darstellung (40), insbesondere das Balkendiagramm (50), eine Skala (151) umfasst, die die Gasvolumina darstellt, und einen beweglichen Cursor (152), der das während jeder einer Insufflationsphase insufflierte Volumen darstellt.

7. Gerät nach Anspruch 1, **dadurch gekennzeichnet, dass** der Anzeigebildschirm (20) dazu ausgestaltet ist, während einer Exsufflationsphase nach einer Insufflationsphase des Patienten, weiterhin das insufflierte Gasvolumen (Vi) anzuzeigen, das während der Insufflationsphase insuffliert worden ist, bevorzugt während einer Exsufflationsphase und einer Pausenphase nach der Insufflationsphase.

8. Gerät nach Anspruch 1, **dadurch gekennzeichnet, dass** die Ansteuerungsmittel (16) dazu ausgestaltet sind, die Anzeige des insufflierten Gasvolumens (Vi) zu Beginn jeder zusätzlichen Insufflationsphase zu reinitialisieren.

9. Gerät nach Anspruch 1, **dadurch gekennzeichnet, dass** der Anzeigebildschirm (20) ferner dazu ausgestaltet ist, ferner eine Anzahl (51) von ausgeführten Insufflationszyklen anzuzeigen.

10. Gerät nach Anspruch 1, **dadurch gekennzeichnet, dass** der Anzeigebildschirm (20) ferner dazu ausgestaltet ist, während mindestens einer Gasinsufflationsphase eine Videoanimation (30, 31) anzuzeigen, die eine grafische Darstellung (30) einsetzt, die einen Patienten (31) symbolisiert, und in der die Form, das Erscheinungsbild und/oder die Abmessungen der grafischen Darstellung (30, 31) in Korrelation mit dem Gasvolumen, das von der Turbine (2) an den Patienten während der mindestens einen Insufflationsphase abgegeben wird, variieren.

11. Gerät nach Anspruch 1, **dadurch gekennzeichnet, dass** die Ansteuerungsmittel (16) dazu ausgestaltet sind, die Turbine (1) zu steuern, um bei dem Patienten während mehrerer aufeinander folgender anfänglicher Insufflationsphasen Gas mit einem gegebenen Volumenstrom zu insufflieren, bis ein vorab festgelegter maximaler Druck oder eine gegebene maximale Einatemdauer erreicht ist.

12. Gerät nach Anspruch 1, **dadurch gekennzeichnet, dass** es Mittel zum Regeln einer Insufflationssitzungsgesamtdauer umfasst, die mehrere zusätzliche Insufflationsphasen umfasst, bevorzugt einer Insufflationssitzungsgesamtdauer von 5 bis 30 Minuten.

13. Gerät nach einem der Ansprüche 4 oder 6, **dadurch gekennzeichnet, dass** das Balkendiagramm (50) eine Skala (151) umfasst, die die gegebenen Gasvolumina in Prozent (%) im Verhältnis zum Zielvolumen (Vc) darstellt, bevorzugt Werte in Prozent (%) zwischen -15 % und +15 %, wobei das Zielvolumen Vc dem Wert 0 % entspricht.

14. Gerät nach einem der Ansprüche 6 oder 13, **dadurch gekennzeichnet, dass** sich der bewegliche Cursor (152) relativ zu der Skala (151) des Balkendiagramms (50) bewegt, um das insufflierte Gasvolumen im Verhältnis zum Zielvolumen (Vc) anzugeben.

15. Gerät nach Anspruch 1, **dadurch gekennzeichnet, dass** die Ansteuerungsmittel (16) dazu ausgestaltet sind, das Ablaufen der angezeigten Videoanimation nach Aktivierung von Verarbeitungsstartmitteln mit Gasinsufflation durch den Benutzer oder nach Detektion eines Beginns einer Einatemphase des Patienten durch Einatemdetektionsmittel zu beginnen.

## Claims

1. Cough assist device (10) for performing at least one insufflation of gas to a patient, comprising:
- a motorized turbine (1) in fluidic communication with a gas circuit (4, 5, 6) comprising an insufflation line (4),
- a display screen (20), and
- control means (16) configured to control the turbine (1) and/or a display on the display screen (20),
**characterized in that** the control means (16) are additionally configured to:
a) control the turbine (1) to insufflate gas to the patient during a plurality of successive initial insufflation phases,
b) determine the gas volumes (Vi) insufflated to the patient during said plurality of successive initial insufflation phases,
c) determine at least one mean gas volume (Vm) on the basis of the gas volumes (Vi) insufflated during said plurality of successive initial insufflation phases,
d) determine or set a target gas volume (Vc) to be administered to the patient on the basis of the mean gas volume (Vm), such that: Vc ≥ Vm,
e) after having determined or set the target gas volume (Vc), control the turbine (1) to provide gas to the patient, during at least one additional insufflation phase following said initial insufflation phases, and
f) control a display on the display screen (20), during each additional insufflation phase, of at least the target gas volume (Vc) and the insufflated gas volume (Vi) administered to the patient during each additional insufflation phase.

2. Device according to Claim 1, **characterized in that** the control means (16) are configured to control the turbine (1) to insufflate gas to the patient during 2 to 10 successive initial insufflation phases.

3. Device according to Claim 1, **characterized in that** in step f), the display screen (20) is configured to display a graphical representation (40) of gas volume comprising at least the insufflated gas volume (Vi) and the target volume (Vc).

4. Device according to Claim 3, **characterized in that** the graphical representation (40) comprises a graph bar (50) giving at least the insufflated gas volume (Vi) and the target volume (Vc).

5. Device according to Claim 1, **characterized in that** in step f), the display screen (20) is additionally configured to display one or more numerical values of insufflated gas volume (Vi) and/or target volume (Vc).

6. Device according to either of Claims 3 and 4, **characterized in that** the graphical representation (40), in particular the graph bar (50), displayed on the display screen (20) comprises a graduated scale (151) representative of the gas volumes and a mobile cursor (152) representative of the insufflated volume, during each insufflation phase.

7. Device according to Claim 1, **characterized in that** the display screen (20) is configured to continue to display, during an exsufflation phase following an insufflation phase of the patient, the insufflated gas volume (Vi) having been insufflated during said insufflation phase, preferably during an exsufflation phase and a pause phase following said insufflation phase.

8. Device according to Claim 1, **characterized in that** the control means (16) are configured to reset the display of the insufflated gas volume (Vi) at the start of each additional insufflation phase.

9. Device according to Claim 1, **characterized in that** the display screen (20) is additionally configured to also display a number (51) of insufflation cycles performed.

10. Device according to Claim 1, **characterized in that** the display screen (20) is additionally configured to also display a video animation (30, 31) implementing a graphical representation (30) symbolizing a patient (31) during at least one gas insufflation phase, and in which the shape, the appearance and/or the dimensions of said graphical representation (30, 31) vary in correlation with the volume of gas provided by the turbine (2) to the patient, during said at least one insufflation phase.

11. Device according to Claim 1, **characterized in that** the control means (16) are configured to control the turbine (1) to insufflate gas to the patient during a plurality of successive initial insufflation phases at a given flow rate until a preset maximum pressure or a given maximum inhalation duration is obtained.

12. Device according to Claim 1, **characterized in that** it comprises means for adjusting a total insufflation session duration comprising a plurality of additional insufflation phases, preferably a total insufflation session duration of 5 to 30 minutes.

13. Device according to either of Claims 4 and 6, **characterized in that** the graph bar (50) comprises a graduated scale (151) representative of the gas volumes given as percentages (%) relative to the target volume (Vc), preferably values as percentages (%) of between - 15% and +15%, the target volume Vc corresponding to the value of 0%.

14. Device according to either of Claims 6 and 13, **characterized in that** the mobile cursor (152) moves relative to the graduated scale (151) of the graph bar (50) so as to indicate the insufflated gas volume relative to the target volume (Vc).

15. Device according to Claim 1, **characterized in that** the control means (16) are configured to start the playing of the video animation displayed after activation by the user of means for starting treatment with gas insufflation or after detection of a start of inspiratory phase of the patient by inhalation detection means.
